## Europäisches Patentamt
## European Patent Office
## Office européen des brevets

(19)

(11) Publication number: **0 099 220**
**A1**

(12) # EUROPEAN PATENT APPLICATION

(21) Application number: **83303874.8**

(22) Date of filing: **04.07.83**

(51) Int. Cl.³: **A 61 M 25/00**
**F 16 L 11/12**

(30) Priority: **05.07.82 GB 8219389**

(43) Date of publication of application:
**25.01.84 Bulletin 84/4**

(84) Designated Contracting States:
**DE FR GB IT**

(71) Applicant: **Longmore, Donald Bernard**
**97 Chertsey Lane**
**Staines Middlesex(GB)**

(72) Inventor: **Longmore, Donald Bernard**
**97 Chertsey Lane**
**Staines Middlesex(GB)**

(74) Representative: **Simpson, Ronald Duncan Innes et al,**
**A.A.Thornton & Co. Northumberland House 303-306**
**High Holborn**
**London WCIV 7LE(GB)**

(54) **Plastics tubing.**

(57) Flexible tubing comprising a main wall portion (8) of Polyvinyl Chloride (PVC) is lined internally with a thin coating (9) of silicone rubber which acts as a barrier against migration of plasticizers and/or other constituents of the PVC material into the interior of the tubing.

FIG. 1.

EP 0 099 220 A1

- 1 -

## Plastics tubing

This invention is concerned with plastics tubing and its manufacture.

Plastics tubing, in particular flexible PVC tubing, is used widely in many industries including applications in which contamination of fluids flowing through the tubing by toxic contaminants is unacceptable or highly undesirable. Examples of such applications are in brewing, biological and chemical processes, and especially in the field of medicine. Although other materials are available, polyvinyl chloride (PVC) tubing is most popular for medical use because it is clear and it is substantially cheaper.

As a necessity, all PVC tubing currently available including that of medical grade, includes plasticisers, generally dethyl-hexyl- phthalate (D.E.H.P.) which is known to be very toxic, or in other cases Trimellitate which is less toxic but 5% more expensive. It is also known that plasticisers, fillers or other additives included in PVC material tend to seep from the surface of the material and a fluid flowing over the comparatively rough inside surface of an extruded PVC tube may easily become contaminated with the additives. D.E.H.P. is frequently found in the blood of patients on dialysis. Furthermore, the toxity of PVC is actually increased if it is subject to sterilisation by gamma radiation or ethylene oxide gas, which are commonly used methods.

Thus the use of PVC tubing including toxic additives is potentially dangerous in medical equipment.

The present invention seeks to provide a solution to the above problem associated with PVC tubing and in accordance with the invention there is provided flexible tubing comprising a main wall portion of polyvinyl chloride (PVC), characterized in that a thin continuous layer of silicone rubber is firmly bonded to the whole of the inner surface of the main wall portion.

The silicone rubber material, e.g. polydimethylsiloxane, and is preferably bonded to the PVC by a direct chemical linking obtained by applying the silicone to the PVC during extrusion of the latter. In this way a strong bond between the material is achieved through a co-polymerisation of the PVC and silicone layers at their interface.

The silicone layer may be very thin, e.g. only a few molecules or microns thick, so that the composite tubing can be substantially transparent, and still provide an effective barrier to prevent toxic additives included in the PVC contaminating fluid flowing through the tubing. The strength of the bonding between the material is believed to be responsible for the efficiency of the barrier provided by the thin silicone layer.

The invention also provides a method of making a composite plastics tubing, including the step of forming a main wall portion of the PVC, characterized in that a thin layer of silicone rubber is then formed on the whole of the inner surface of the main wall portion.

In a preferred manufacturing process to ensure a good chemical bond between the PVC and silicone layers a partially cured solution of polydimethysiloxane is atomised and sprayed onto the inside of the tube as it leaves the extruder outlet and before the polymerisation of the PVC is completed so that a co-polymerisation of the two layers is achieved. Applying the silicone to the tube at a distance of about 0.2 to 0.5 cms from the outlet of the extruder has been found suitable.

The silicone (polydimethylsiloxane) may be dissolved in a solvent having a boiling point up to 170°C, e.g. toluene and preferably crude n-hexane fractions such as ESSO 10 boiling range 50° to over 110°C.

According to the invention there is provided also an apparatus for making the composite tubing, comprising an extrusion press having an annular extrusion outlet, characterized by a spray nozzle positioned close to the extrusion outlet downstream thereof and operative to direct fine spray of silicone rubber in solution against the interior surface of the main wall portion as it leaves the extrusion outlet.

In a preferred form, the nozzle comprises a plurality of outlet orifices spaced apart around the nozzle and inclined rearwardly towards the extrusion outlet. The orifices may be staggered in the axial direction and extend spirally outwardly from a central passageway with a view to increasing turbulence within the tube and hence ensuring a uniform layer of silicone deposited on the interior of the extruded tube.

A full understanding of the invention will be had from the following detailed description given with reference to the accompanying drawing in which:

Figure 1 is a schematic illustration of an extruder embodying the invention;

Figure 2 is an enlarged scale section taken along the line II-II in Figure 1; and

Figure 3 is a view similar to Figure 2 and showing a modified nozzle.

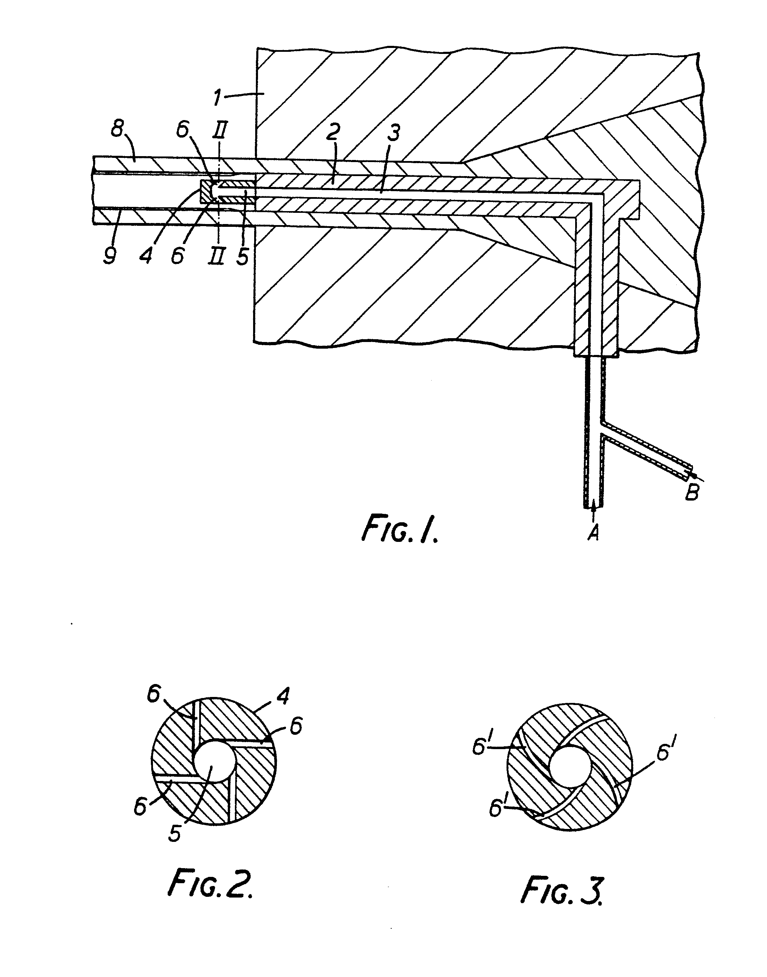

The extruder shown in Figure 1 is illustrated schematically in axial cross-section. It comprises an annular extrusion outlet defined between an outer die or body 1 and an inner core 2. The core has an axial air duct 3 the inlet end of which is connected to a source (A) of compressed air and at (B) to an atomiser (not shown), for example of the ultrasonic type which atomises a solution of polydimethylsiloxane in toluene. Alternatively, a so-called airless spray gun may be employed. Fixed to the end of the core 2 and communicating with the air duct 3 is a nozzle 4 made of metal or plastics. The nozzle has a central passage 5 which is closed at its outer end and from which radiate several orifices 6 which are inclined rearwardly towards the extrusion outlet. The outlets from the nozzle are preferably located at a distance of about 0.2. cms to 0.5 cms from the extruder outlet. As shown in Figure 1 adjacent orifices may be staggered along the nozzle to enable a greater number of orifices to be located around the nozzle periphery for producing a more uniform spray of silicone solution at the interior of the tube. The nozzle surface is spaced from the inside of the extruded tube by a clearance preferably not less than 1/5 of the diameter of the extrusion.

In operation the extruder extrudes a continuous tube 8 of PVC material. Compressed air and atomised partially cured polydimethylsiloxane solution are supplied to the air duct 3 and a spray of the solution is directed towards the inside of the extruded tube through the orifices 6 of the nozzle 4. The polydimethylsiloxane is deposited on the inside of the tube as a continuous substantially uniform film 9 and its polymerisation is completed at the same time as that of the PVC so that a strong bond is obtained between the two layers by virtue of their co-polymerisation. To aid the chemical bonding it is of advantage to allow the composite tubing product to run a little longer than usual before cooling. The nozzle orifices 6 may be adapted to enhance the atomisation of the silicone in order to obtain a more homogenous spray and hence a more uniform coating around the periphery of the PVC tube. In the modified nozzle shown in Figure 3 the orifices 6' extend spirally for producing a swirling spray also with a view to improving

- 5 -

the uniformity of the silicone layers.

The solvent vapour which is retained in the tubing can be removed by blowing through with filtered air and it can be recovered for re-use if desired.

It is possible that the need for subsequent sterilisation of the composite tubing may be eliminated by incorporating a bacterial filter at the inlet of the air duct.

Claims

1.      Flexible tubing comprising a main wall portion of polyvinyl chloride (PVC), characterized in that a thin continuous layer of silicone rubber is firmly bonded to the whole of the inner surface of the main wall portion.

2.      Flexible tubing according to claim 1, characterized in that the silicone rubber layer has a thickness of between several molecules and several microns.

3.      Flexible tubing according to claim 1 or 2, characterized in that the silicone rubber layer is bonded to the main wall portion by a direct chemical bond.

4.      Flexible tubing according to claim 3, characterized in that the silicone rubber layer has been formed in situ on the inner surface of the main wall portion during extrusion thereof.

5.      A method of making a composite  plastics tubing, including the step of forming a main wall portion of PVC, characterized in that a thin layer of silicone rubber is then formed on the whole of the inner surface of the main wall portion.

6.      A method according to claim 5, wherein the main wall portion is formed by extrusion of PVC material, and the said layer is formed by deposition of silicone rubber as a spray against the inner surface of the main wall portion as it leaves the extrusion outlet by which it is formed.

7.      Apparatus for use in performing the method of claim 5, comprising an extrusion press having an annular extrusion outlet, characterized by a spray nozzle, positioned close to the extrusion outlet downstream thereof and operative to direct fine spray of silicone rubber in solution against the interior surface of the main wall portion as it leaves the extrusion outlet.

8.     Apparatus according to claim 7, characterized in that the spray nozzle comprises a plurality of outlet orifices spaced apart around the outlet nozzle and inclined rearwardly towards the extrusion outlet.

0099220

1/1

FIG. 1.

FIG. 2.

FIG. 3.

**European Patent Office**

**EUROPEAN SEARCH REPORT**

Application number

EP 83 30 3874

## DOCUMENTS CONSIDERED TO BE RELEVANT

| Category | Citation of document with indication. where appropriate, of relevant passages | Relevant to claim | CLASSIFICATION OF THE APPLICATION (Int. Cl. 3) |
|---|---|---|---|
| X | DE-B-1 491 682 (DOW CORNING) * Column 1, line 36 - column 2, line 24 * | 1-3 | A 61 M 25/00 F 16 L 11/12 |
| A | US-A-2 355 756 (SMITH) * Page 1, column 1, lines 40-49; page 2, column 1, lines 2-6; figure 2 * | 1,4-8 | |
| A | US-A-4 211 741 (OSTOICH) * Column 1, lines 35-51; figure 3 * | 1,4 | |
| A | GB-A-1 110 944 (PAXMAN COOLER MANUFACTURING) | | |

|  |  |
|---|---|
|  | TECHNICAL FIELDS SEARCHED (Int. Cl. 3) |
|  | A 61 M 25/00 F 16 L 11/00 B 29 F 3/00 |

The present search report has been drawn up for all claims

| Place of search | Date of completion of the search | Examiner |
|---|---|---|
| BERLIN | 20-09-1983 | SCHLABBACH M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document. but published on. or after the filing date
D : document cited in the application
L : document cited for other reasons

& : member of the same patent family, corresponding document

EPO Form 1503 03 82